# FASCICULE DE BREVET EUROPEEN

(11) **EP 0 704 432 B1**
(45) Date de publication et mention de la délivrance du brevet: **07.01.1998**
(21) Numéro de dépôt: 95402075.6
(22) Date de dépôt: 14.09.1995
(51) Int. Cl.: C07C 381/02, C07C 319/24, C07C 323/16

(54) **Procédé de synthèse de disulfures dissymétriques**
Verfahren zur Herstellung von unsymmetrischen Disulfiden
Process for the preparation of asymmetric disulphides

(30) Priorité: 29.09.1994 FR 9411609
(43) Date de publication de la demande: 03.04.1996
(73) Titulaire: SOLLAC S.A., 92800 Puteaux (FR)
(72) Inventeur: Hiver, Patricia Nathalie, F-57070 Metz Borny (FR); Schwendimann, Christian René, F-57070 Metz (FR); Dicko, Amadou, F-57070 Metz-Vallieres (FR); Paquer, Daniel André, F-54500 Vandoeuvre (FR)
(74) Mandataire: Ventavoli, Roger

(56) Documents cités:
- ANGEWANDTE CHEMIE. INTERNATIONAL EDITION, vol. 6, no. 6, 1967 WEINHEIM, DE, pages 544-553, H. DISTLER: 'The chemistry of Bunte salts'

## Description

L'invention concerne le domaine de la synthèse de molécules organiques disulfurées, et plus particulièrement de disulfures dissymétriques, notamment benzyliques.

On connaît dans la littérature de nombreuses méthodes de synthèse de disulfures dissymétriques.

L'une de ces méthodes consiste à réaliser cette synthèse par substitution nucléophile d'un thiolate du type R - S^{⊖} K^{⊕} sur un alkyl thiosulfate (sel de Bunte) du type R'- S₂O₃^{⊖} Na^{⊕} , pour obtenir un composé R - S₂ - R'. Le sel de Bunte est lui-même obtenu par réaction de thiosulfate de sodium Na₂S₂O₃ avec le dérivé bromé R' - Br. Cette préparation est avantageuse en ce qu'elle ne nécessite qu'un nombre d'étapes réactionnelles relativement réduit, et en ce que l'un des principaux réactifs (le thiosulfate de sodium) est un produit courant et bon marché.

Cette méthode ne peut, toutefois, être appliquée lorsqu'on désire synthétiser un composé où le radical R' comprend un noyau aromatique qui n'est, par ailleurs, substitué que par une fonction hydroxylique (par exemple -CH₂-Ar-OH). En effet, les dérivés bromés du type de 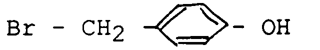 ne peuvent être synthétisés ainsi puisque la réaction de bromation du dérivé 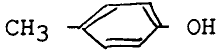 est empêchée par la présence du groupement hydroxyle. Cette bromation ne serait possible que si le noyau aromatique était substitué par des groupes alkyles, placés en position ortho par rapport à l'hydroxyle (voir le document FR-A-2 629 817).

On peut penser à synthétiser ce dérivé bromé 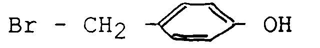 en réalisant tout d'abord une protection de l'hydroxyle par une fonction ester ou par un groupement triméthyl silane pour obtenir 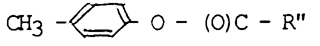 ou 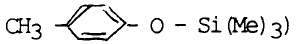 puis en réalisant la bromation de ce dérivé pour obtenir 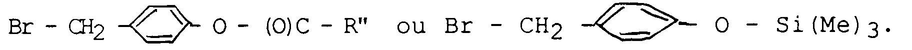 On forme ensuite le sel de Bunte, puis le disulfure, et on réalise enfin la déprotection de la fonction -OH pour parvenir au composé recherché (voir le document "Préparation de sulfures phénoliques, étude en RMN C13", Sulfur Letters, Vol. 8(2), pp. 115-118). Mais la synthèse du sel de Bunte ne peut être poursuivie de manière satisfaisante à l'aide de thiosulfate de sodium dans un milieu au moins partiellement aqueux (par exemple: méthanol-eau). Dans le cas où la protection du groupement hydroxyle a été assurée par une fonction ester, on assiste à une réaction de solvolyse, et la protection silane est en grande partie détruite dans ce milieu aqueux.

On ne connaît donc actuellement pas de méthode qui permettrait de préparer avec un rendement satisfaisant par l'intermédiaire d'un sel de Bunte des disulfures benzyliques dissymétriques où le noyau aromatique serait substitué par un groupement hydroxyle, avec des atomes d'hydrogène en position ortho par rapport à ce groupement hydroxyle.

Le but de l'invention est de rendre possible une telle préparation.

L'invention a pour objet un procédé de synthèse de disulfures dissymétriques R - S₂ - R', R et R' étant des radicaux carbonés fonctionnalisés ou non, selon lequel, à partir d'un composé R' - H, on prépare un composé du type R' - Br, puis on prépare un sel de Bunte R' - S₂O₃^{⊖} Na^{⊕} par réaction dudit composé R' - Br avec du thiosulfate de sodium, puis on fait réagir ledit sel de Bunte avec un thiolate du type R - S^{⊖} K^{⊕} pour former ledit disulfure, caractérisé en ce que la préparation dudit sel de Bunte est effectuée dans du diméthylsulfoxyde.

Ce procédé concerne plus particulièrement les cas où le radical R' comporte un noyau aromatique substitué par un groupement hydroxyle, groupement qu'il est nécessaire de protéger avant la préparation du composé R' - Br et de déprotéger pendant ou après la réaction du sel de Bunte avec le thiolate.

Comme on l'aura compris, l'invention consiste à réaliser la préparation du sel de Bunte non dans un milieu partiellement aqueux comme à l'habitude, mais dans du diméthylsulfoxyde, ce qui permet de préserver la protection du groupement hydroxyle, et ainsi d'assurer un rendement satisfaisant à la réaction de préparation des composés recherchés.

Les inventeurs se sont en effet rendus compte que le remplacement des solvants aqueux usuellement employés dans la préparation des sels de Bunte par du diméthylsulfoxyde (qui sera abrégé en DMSO dans la suite de la description) permettait de conserver intacte la protection du groupement hydroxyle pendant cette préparation.

On parvient ainsi à lever tous les obstacles à l'obtention finale d'un disulfure benzylique dont le noyau aromatique est substitué par un groupement hydroxyle et ne comporte pas de substituants en position ortho par rapport à cet hydroxyle. On citera plus loin des applications possibles pour ces nouveaux composés.

Le DMSO est un solvant qui autorise une dissociation totale du thiosulfate de sodium, sans qu'il soit besoin d'y adjoindre un certaine quantité d'eau. On notera que la présence d'eau lors de la préparation du sel de Bunte était jusqu'à présent préconisée par la littérature. Le caractère fortement polaire du DMSO lui permet de se substituer avantageusement à l'eau pour obtenir la dissociation totale du thiosulfate de sodium. Et lors de l'étape réactionnelle suivante, durant laquelle on introduit le dérivé bromé 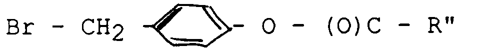 ou 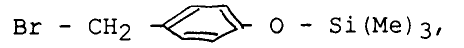 l'absence d'eau dans le milieu réactionnel permet d'éviter la destruction de la protection silane ou la solvolyse du composé ester. Cela est dû au caractère aprotique du DMSO. Par ailleurs, la dissociation totale du thiosulfate de sodium est favorable à un bon rendement de l'obtention du sel de Bunte.

Le sel de Bunte R' - S₂O₃^{⊖} Na^{⊕} (où R' est un groupement 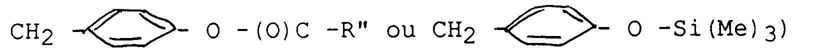 ainsi obtenu est ensuite, de manière connue, mis en présence d'un thiolate du type R - S^{⊖} K^{⊕} pour former un disulfure R - S₂ - R'.

Si l'objectif final de la synthèse est l'obtention d'un disulfure benzylique dont le noyau aromatique est substitué par un groupement hydroxyle, il faut enfin réaliser la "déprotection" de la fonction hydroxylique. Dans le cas d'une protection ester, cette déprotection est effectuée par saponification de l'ester, et doit être réalisée au cours d'une étape réactionnelle séparée, après isolation du disulfure. En revanche, dans le cas d'une protection silane, la déprotection a lieu simultanément à la formation du disulfure. Cette protection se révèle donc particulièrement avantageuse par rapport à la protection ester, puisqu'elle permet d'économiser une étape réactionnelle, d'où un gain de temps et un meilleur rendement global de la synthèse. Les réactifs utilisés pour la protection silane sont également moins coûteux que ceux utilisés pour la protection ester. Toutefois, la protection silane nécessite une plus grande rigueur dans la réalisation des réactions, car le composé bromé ainsi protégé est très instable en présence d'air ou d'eau.

Deux exemples de synthèses selon l'invention vont à présent être décrits. Ils visent tous deux à l'obtention, à partir de 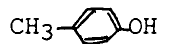 (p. crésol), d'un composé du type 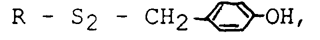 et se distinguent par le type de protection du groupement hydroxyle auquel ils font appel.

### Exemple 1 : utilisation d'une protection ester.

### 1) Protection de la fonction hydroxylique

Cette étape, comme on l'a dit, est indispensable pour préparer ensuite le composé bromé par réaction radicalaire. On utilise à cet effet l'ester benzoïque qui permet une purification aisée des produits obtenus.

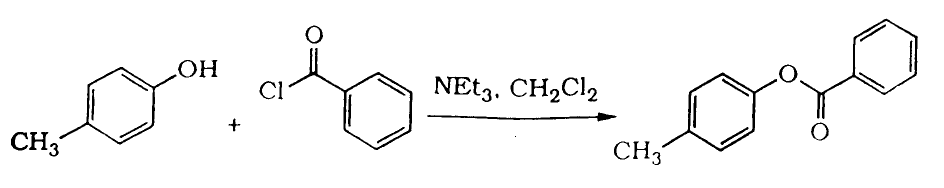

Dans un tricol de 500 ml muni d'un réfrigérant, d'une ampoule à brome et d'une agitation magnétique, on introduit 10,8 g (0,1 mole) de p. crésol, 100 ml de triéthylamine et 200 ml de dichlorométhane.

Après homogénéisation, on additionne très lentement 14 g (0,1 mole) de chlorure de benzoyle. On laisse ainsi sous agitation à température ambiante pendant 1 heure. Le sel formé est ensuite filtré sur büchner et le solvant évaporé. On obtient des cristaux blancs de méthylène aromatique qui sont recristallisés dans du cyclohexane.

### 2) Bromation du méthylène aromatique

Elle s'effectue par réaction radicalaire, donc en milieu totalement anhydre (puisqu'un groupement hydroxylique libre inhibe toute réaction radicalaire).

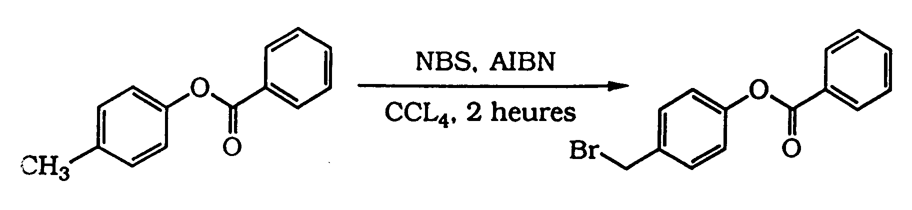

Dans un tricol de 250 ml muni d'un réfrigérant, d'une ampoule à brome et d'une agitation magnétique, on dissout 15 g (0,07 mole) du méthylène aromatique obtenu à l'étape 1) dans 150 ml de tétrachlorure de carbone anhydre. On additionne ensuite lentement 13 g (0,073 mole) de N-bromo succinimide (NBS) et 1 g d'Azo (bis) isobutyronitrile (AIBN). On porte ensuite à reflux pendant 2 heures. En fin de réaction, la succinimide formée est filtrée sur büchner et le solvant est évaporé sous pression réduite.

### 3) Formation du disulfure

Selon l'invention, cette étape utilise du DMSO comme solvant pour la préparation des sels de Bunte.

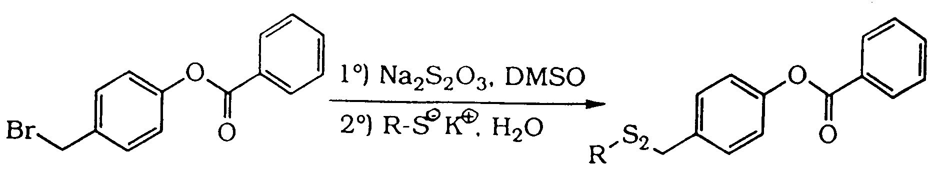

Elle s'effectue en trois étapes :

### a) Synthèse du sel de Bunte

Dans un tricol de 500 ml muni d'un réfrigérant, d'une ampoule à brome et d'une agitation magnétique, on introduit 5 g (0,02 mole) de thiosulfate de sodium et 200 ml de DMSO. On porte le mélange à 80°C jusqu'à dissolution du thiosulfate.

On laisse revenir à température ambiante et on ajoute goutte à goutte un équivalent de dérivé bromé de l'étape 2). On porte le mélange à 80°C pendant 4 heures. On obtient ainsi un alkyl thiosulfate

### b) Synthèse du thiolate

Parallèlement à l'étape a), dans un tricol de 100 ml muni d'un réfrigérant, d'une ampoule à brome et d'une agitation magnétique, on introduit 1,2 g (0,02 mole) de potasse et on ajoute 20 ml d'eau.

On ajoute ensuite un équivalent de mercaptan R - SH, et on porte le mélange à 50 °C pendant 30 minutes. R est un groupement carboné quelconque, par exemple, du type tertiobutyle, benzyle, dodécyle, tertiododécyle, fonctionnalisé ou non (dans la mesure ou cette fonctionnalisation n'intervient pas défavorablement sur les réactions mises en jeu par cette synthèse).

On forme ainsi un mercaptate de potassium R - S^{⊖} K^{⊕}.

### c) Synthèse du disulfure

On ajoute lentement la solution aqueuse du mercaptate dans la solution contenant l'alkyl thiosulfate, et on laisse sous agitation durant 1 heure. Après refroidissement, on ajoute du chloroforme et on lave la solution plusieurs fois à l'eau pour éliminer le DMSO.

On sèche ensuite la phase organique sur du sulfate de sodium et on évapore le solvant sous vide. Le rendement de la réaction est de l'ordre de 60 %.

### 4) Déprotection de la fonction hydroxylique

Elle consiste en une saponification de la fonction ester:

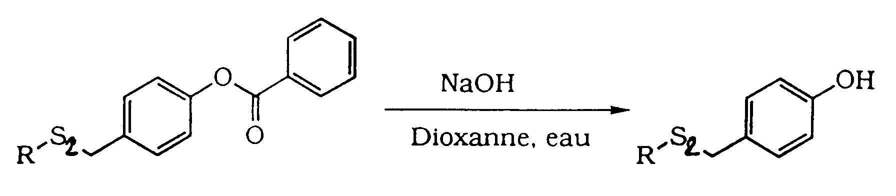

Dans un tricol de 250 ml muni d'un réfrigérant, d'une ampoule à brome et d'une agitation magnétique, on introduit 1,1 g (0,02 mole) de soude dans une solution de 50 ml de dioxanne et 50 ml d'eau.

Après dissolution, on ajoute un équivalent du disulfure à déprotéger obtenu lors de l'étape 3), et on laisse ainsi sous agitation pendant 3 heures. On évapore ensuite le dioxanne sous vide, puis on extrait le résidu au dichlorométhane. On sèche la phase organique au sulfate de sodium et on évapore le solvant sous vide. Le rendement de la réaction varie généralement de 50 à 60 %, en fonction de la nature du radical R.

### Exemple 2 : utilisation d'une protection silane

### 1) Protection de la fonction hydroxylique

L'agent de silylation utilisé dans cet exemple est l'hexaméthyldisilazane (HMDS).

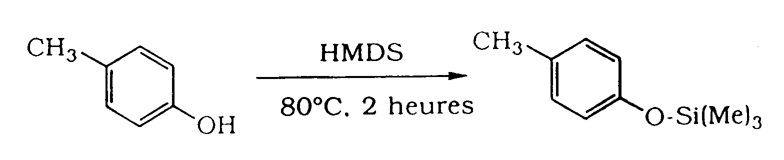

Dans un tricol de 250 ml muni d'un réfrigérant et d'une ampoule à brome, on introduit 10,8 g (0,1 mole) de p.crésol.

On additionne ensuite lentement 16,1 g (0,1 mole) de HMDS et on porte le mélange à reflux pendant 2 heures à 80°C. L'excès de HMDS est évaporé sous vide, et le produit est distillé sous pression réduite.

### 2) Bromation du méthylène aromatique

Elle s'effectue de manière identique à la réaction correspondante à l'exemple 1.

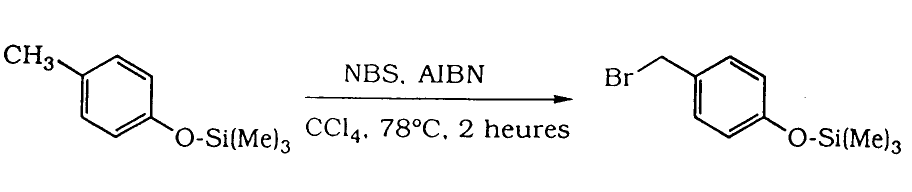

On obtient après évaporation du solvant sous vide un liquide jaune instable susceptible de se décomposer à l'air. Des précautions particulières doivent donc être prises pour sa conservation, telles qu'un stockage sous gaz inerte.

### 3) Formation du disulfure

Elle s'effectue de manière identique à la réaction correspondante de l'exemple 1, à cette différence près que l'on obtient directement un produit dont la fonction hydroxylique est déprotégée, donc sans nécessité d'isoler un produit intermédiaire qu'il faudrait ensuite déprotéger.

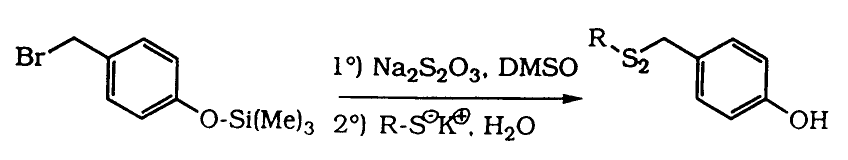

Les disulfures dissymétriques benzyliques hydroxylés obtenus par cette synthèse peuvent avantageusement être utilisés comme produits intermédiaires pour synthétiser des composés présentant à la fois une fonction disulfure et une autre fonction qui viendrait se substituer à la fonction hydroxylique.

Comme exemples de tels composés, on peut citer ceux du type : 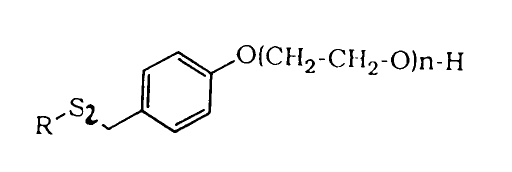 obtenus par polyéthoxylation des produits intermédiaires précédents. Ils peuvent être utilisés comme additifs à des huiles de laminage, le motif soufré leur procurant des propriétés anti-usure et extrême pression, et le motif polyéthoxylé constituant un pôle hydrophile à propriétés tensio-actives.

Bien entendu, l'invention n'est pas limitée aux exemples qui viennent d'être décrits. En particulier, l'utilisation du DMSO comme solvant lors de la préparation du sel de Bunte aboutissant au disulfure peut être appliquée lors de la synthèse de tout disulfure dissymétrique R - S₂ - R' et pas seulement des disulfures dissymétriques benzyliques

## Revendications

1. Procédé de synthèse de disulfures dissymétriques R - S₂- R', R et R' étant des radicaux carbonés fonctionnalisés ou non, selon lequel, à partir d'un composé R' - H on prépare un composé du type R' - Br, puis on prépare un sel de Bunte R' - S₂O₃^{⊖} Na^{⊕} par réaction dudit composé R' - Br avec du thiosulfate de sodium, puis on fait réagir ledit sel de Bunte avec un thiolate du type R - S^{⊖} K^{⊕} pour former ledit disulfure, caractérisé en ce que la préparation dudit sel de Bunte est effectuée dans du diméthylsulfoxyde.

2. Procédé selon la revendication 1, caractérisé en ce que le radical R' comprend un noyau aromatique.

3. Procédé selon la revendication 2, caractérisé en ce que ledit noyau aromatique est substitué par un groupement hydroxyle, en ce que, préalablement à la préparation du composé R' - Br, on réalise une protection du groupement hydroxyle, et en ce que, après la réaction, on réalise la déprotection dudit groupement hydroxyle.

4. Procédé selon la revendication 3, caractérisé en ce que ladite protection est effectuée par une fonction ester, et en ce que ladite déprotection est effectuée par saponification de ladite fonction ester.

5. Procédé selon la revendication 3, caractérisé en ce que ladite protection est effectuée par une fonction silane.

## Claims

1. Process for the synthesis of asymmetric disulphides R-S₂-R', R and R' being functionalized or non-functionalized hydrocarbon radicals, according to which a compound of the R'-Br type is prepared from a compound R'-H, a Bunte salt R'-S₂O₃^{⊖} Na^{⊕} is then prepared by reaction of the said compound R'-Br with sodium thiosulphate, and then the said Bunte salt is reacted with a thiolate of the R-S^{⊖} K^{⊕} type in order to form the said disulphide, characterized in that the preparation of the said Bunte salt is carried out in dimethyl sulphoxide.

2. Process according to Claim 1, characterized in that the R' radical comprises an aromatic ring.

3. Process according to Claim 2, characterized in that the said aromatic ring is substituted by a hydroxyl group, in that, prior to the preparation of the compound R'-Br, protection of the hydroxyl group is carried out, and in that, after the reaction, deprotection of the said hydroxyl group is carried out.

4. Process according to Claim 3, characterized in that the said protection is carried out by an ester functional group and in that the said deprotection is carried out by saponification of the said ester functional group.

5. Process according to Claim 3, characterized in that the said protection is carried out by a silane functional group.

## Patentansprüche

1. Verfahren zur Herstellung asymmetrischer Disulfide R - S₂ - R', worin R und R' funktionalisierte oder nichtfunktionalisierte Kohlenstoffradikale sind, bei dem man, ausgehend von einer Verbindung R' - H, eine Verbindung des Typs R' - Br herstellt, worauf man ein Bunte-Salz R' - S₂O₃⁻ Na⁺ durch Reaktion der besagten Verbindung R' - Br mit Natriumthiosulfat herstellt, wonach man das besagte Bunte-Salz mit einem Thiolat des Typs R - S⁻ K⁺ umsetzt, um das besagte Disulfid zu bilden, dadurch gekennzeichnet, dass die Herstellung des besagten Bunte-Salzes in Dimethylsulfoxid durchgeführt wird.

2. Verfahren nach Anspruch 1, dadurch gekennzeichnet, dass das Radikal R' einen aromatische Kern aufweist.

3. Verfahren nach Anspruch 2, dadurch gekennzeichnet, dass der besagte aromatische Kern durch eine Hydroxyl-Gruppe substituiert ist, dass vor der Herstellung der Verbindung R' - Br ein Schutz der Hydroxyl-Gruppe erfolgt, und dass nach der Reaktion eine Freisetzung der besagten Hydroxylgruppe durchgeführt wird.

4. Verfahren nach Anspruch 3, dadurch gekennzeichnet, dass der besagte Schutz durch eine Ester-Funktion erfolgt, und dass die besagte Freisetzung durch Verseifung der besagten Ester-Funktion durchgeführt wird.

5. Verfahren nach Anspruch 3, dadurch gekennzeichnet, dass der besagte Schutz durch eine Silan-Funktion erfolgt.
